# EUROPÄISCHE PATENTSCHRIFT

(11) **EP 0 413 230 B1**
(45) Veröffentlichungstag und Bekanntmachung des Hinweises auf die Patenterteilung: **30.03.1994**
(21) Anmeldenummer: 90115113.4
(22) Anmeldetag: 07.08.1990
(51) Int. Cl.: C07C 309/46

(54) **Nitroanilinsulfonsäuren und Verfahren zur Herstellung von Phenylendiaminsulfonsäuren**
Nitroaniline sulphonic acids and process for preparing phenylenediamino sulphonic acids
Acides nitraniline-sulfoniques et procédé pour la préparation d'acides phénylène-diamine-sulfoniques

(30) Priorität: 16.08.1989 DE 3927068
(43) Veröffentlichungstag der Anmeldung: 20.02.1991
(73) Patentinhaber: BASF Aktiengesellschaft, 67063 Ludwigshafen (DE)
(72) Erfinder: Patsch, Manfred, Dr., D-6706 Wachenheim (DE); Pandl, Klaus, Dr., D-6700 Ludwigshafen (DE)

(56) Entgegenhaltungen:
- EP-A- 0 146 889
- EP-A- 0 285 972
- DE-C- 294 547

## Beschreibung

Die vorliegende Erfindung betrifft neue Nitroanilinsulfonsäuren der Formel I 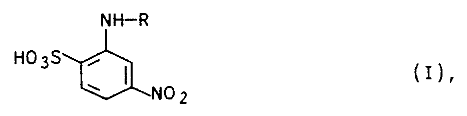
in der R C₁-C₄-Alkyl bedeutet.

Die Herstellung von 4-Nitro-2-aminobenzolsulfonsäure durch Sulfonierung von 3-Nitroanilin mittels Oleum ist in der DE-A-294 547 beschrieben.

Aus der EP-A-146 889 sind ebenfalls Nitroanilinsulfonsäuren bekannt, jedoch weisen diese ein anderes Substitutionsmuster auf.

Aufgabe der vorliegenden Erfindung war es, neue N-alkylsubstituierte Nitroanilinsulfonsäuren bereitzustellen.

Demgemäß wurden die eingangs näher bezeichneten Nitroanilinsulfonsäuren der Formel I gefunden.

Reste R in Formel I sind z.B. Methyl, Ethyl, Propyl, Isopropyl, Butyl, Isobutyl oder sec-Butyl.

Bevorzugt sind solche Nitroanilinsulfonsäuren der Formel I, in der R Methyl oder Ethyl bedeutet.

Die erfindungsgemäßen Nitroanilinsulfonsäuren der Formel I sind wertvolle Zwischenprodukte für die Herstellung von Phenylendiaminsulfonsäuren der Formel II 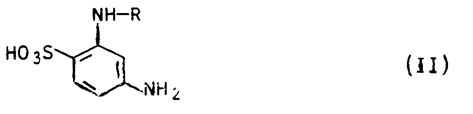
in der R die obengenannte Bedeutung besitzt. Diese wiederum eignen sich vorteilhaft für die Herstellung von wertvollen Kupferformazan-Reaktivfarbstoffen, wie sie beispielsweise in der EP-A-315 045 oder EP-A-315 046 beschrieben sind.

Die erfindunsgemäßen Nitroanilinsulfonsäuren der Formel I werden vorteilhaft erhalten, wenn man Nitroaniline der Formel III 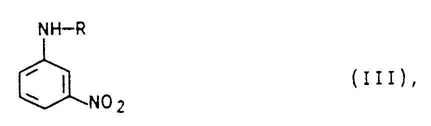
in der R die obengenannte Bedeutung besitzt, mit Oleum behandelt.

Die Sulfonierung der Nitroaniline III erfolgt im allgemeinen mit 20 bis 25 gew.-%igem Oleum bei einer Temperatur von 120 bis 150°C.

Je Gewichtsteil Nitroanilin III verwendet man üblicherweise 0,5 bis 2,0 Gew.-Teile Oleum.

Dabei gibt man zweckmäßig zunächst das Nitroanilin III in konzentrierte Schwefelsäure und fügt anschließend ca. 65 gew.-%iges Oleum zu, bis die obengenannte Konzentration erreicht ist. Nach Erhitzen auf die erfindungsgemäße Temperatur wird das Reaktionsgemisch für einen Zeitraum von 3 bis 8 Stunden bei dieser Temperatur gehalten, dann abgekühlt und auf Eis gegeben. Die erfindungsgemäße Nitroanilinsulfonsäure I fällt als Niederschlag an und wird abgetrennt.

Die erfindungsgemäßen Nitroanilinsulfonsäuren I können nach an sich bekannten Methoden, wie sie beispielsweise in Houben-Weyl, "Methoden der Organischen Chemie" Band 11/1, S. 360 ff, beschrieben sind, zu den Phenylendiaminsulfonsäuren II reduziert werden.

Vorzugsweise führt man eine katalytische Reduktion mittels Wasserstoff durch, wobei man Palladium oder insbesondere Raney-Nickel als Katalysator verwendet. Palladium wird dabei im allgemeinen in Form eines Trägerkatalysators angewandt, wobei als Träger insbesondere Kohle zu nennen ist.

Die katalytische Hydrierung führt man im allgemeinen in wäßrigem Medium bei einer Temperatur von 20 bis 60°C und einem Wasserstoff-Druck von 1,0 bis 6,0 bar durch. Der pH-Wert liegt üblicherweise bei 5 bis 11. Nachdem die Wasserstoffaufnahme beendet ist, wird in der Regel vom Katalysator abfiltriert und das Filtrat angesäuert. Das als Niederschlag anfallende Zielprodukt wird dann abgetrennt.

Eine weitere bevorzugte Reduktionsmethode ist die Umsetzung mit Eisen nach Béchamp.

Die Béchamp-Reduktion führt man im allgemeinen bei einer Temperatur von 50 bis 100°C in saurem Medium, vorzugsweise in Essigsäure durch. Besonders bevorzugt ist eine Verfahrensweise, bei der man in 5 bis 20 gew.-%iger wäßriger Essigsäure arbeitet.

Je Mol Nitroanilinsulfonsäure verwendet man dabei in der Regel 2 bis 20 Grammatom Eisen.

Nach beendeter Reduktion, die üblicherweise 1 bis 5 Stunden in Anspruch nimmt, wird das Reaktionsgemisch auf einen pH-Wert von ca. 8 bis 10 gestellt und anschließend abfiltriert. Das Filtrat wird angesäuert, wobei die Phenylendiaminsulfonsäure als Niederschlag ausfällt und abgetrennt wird.

Die folgenden Beispiele sollen die Erfindung näher erläutern.

### Beispiel 1

132 g 3-Nitro-N-methylanilin wurden bei Raumtemperatur in 240 g konz. Schwefelsäure gegeben, wobei die Temperatur auf 85°C stieg. Nach Zugabe von 138 g Oleum (65 gew.-%ig) wurde auf 130 bis 140°C erhitzt und diese Temperatur 5 Stunden gehalten. Nach dem Abkühlen wurde die Lösung auf 1000 g Eis gegeben und die ausgefallene 5-Nitro-N-methylanilin-2-sulfonsäure wurde abfiltriert.
¹³C-NMR-Daten:
δ (D⁶-DMSO): 149,1 (s), 146,1 (s), 136,4 (s), 128,1 (d), 109,3 (d), 104,1 (d), 30,0 (q)

### Beispiel 2

100 g 5-Nitro-N-methylanilin-2-sulfonsäure wurden in eine siedende Mischung aus 1000 g Wasser, 312 g Eisen und 105 g Essigsäure gegeben und 3 Stunden unter Rückfluß erhitzt. Nach dem Abkühlen wurde mit Natronlauge ein pH-Wert von 8 eingestellt und der resultierende Niederschlag abfiltriert. Das Filtrat wurde mit Salzsäure auf einen pH-Wert von 1,0 gestellt und die ausgefallene 3-(N-Methylamino)anilin-4-sulfonsäure wurde abgesaugt.
¹³C-NMR-Daten:
δ (D⁶-DMSO): 153,8 (s), 150,5 (s), 131,8 (d), 120,2 (s), 106,2 (d), 100,8 (d), 32,5 (q)

### Beispiel 3

144 g 3-Nitro-N-ethylanilin wurden analog Beispiel 1 umgesetzt. Man erhielt 170 g 5-Nitro-N-ethylanilin-2-sulfonsäure.
¹³C-NMR-Daten:
δ (D⁶-DMSO): 148,9 (s), 145,3 (s), 136,5 (s), 128,2 (d), 108,9 (d), 104,1 (d), 37,5 (t), 14,0 (q)

### Beispiel 4

100 g 5-Nitro-N-ethylanilin-2-sulfonsäure wurden analog Beispiel 2 umgesetzt. Man erhielt 85 g 3-(N-Ethylamino)anilin-4-sulfonsäure.
¹³C-NMR-Daten:
δ (D⁶-DMSO): 142,7 (s), 137,7 (s), 128,6 (s und d), 109,1 (d), 104,8 (d), 34,4 (t), 13,5 (q)

### Beispiel 5

23,2 g 5-Nitro-N-methylanilin-2-sulfonsäure wurden in 300 g Wasser gegeben. Dann wurde mit Natronlauge ein pH-Wert von 5,0 bis 5,5 eingestellt, 2 g Raney-Nickel hinzugegeben und unter Normaldruck bei 30°C hydriert. Nach Ende der Wasserstoffaufnahme wurde der Katalysator abfiltriert, der pH-Wert des Filtrats mit Salzsäure auf 0,5 bis 1,0 gestellt und die gefällte 3-(N-Methylamino)anilin-4-sulfonsäure durch Filtration isoliert.

### Beispiel 6

Man verfuhr analog Beispiel 5, verwendete jedoch 24,6 g 5-Nitro-N-ethylanilin-2-sulfonsäure. Man erhielt 21,0 g 3-(N-Ethylamino)anilin-4-sulfonsäure.

## Patentansprüche

1. Nitroanilinsulfonsäuren der Formel I 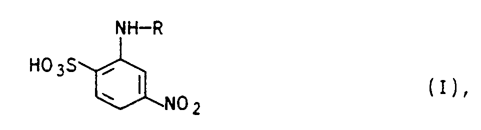 in der R C₁-C₄-Alkyl bedeutet.

2. Verfahren zur Herstellung von Nitroanilinsulfonsäuren gemäß Anspruch 1, dadurch gekennzeichnet, daß man Nitroaniline der Formel III 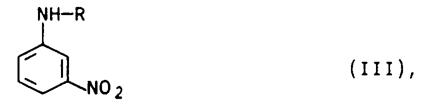 in der R die obengenannte Bedeutung besitzt, mit Oleum behandelt.

## Claims

1. A nitroanilinesulfonic acid of the formula I 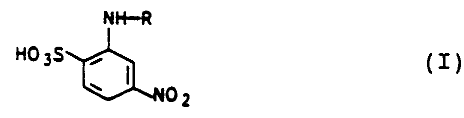 where R is C₁-C₄-alkyl.

2. A process for the preparation of nitroanilinesulfonic acids as claimed in claim 1, which comprises treating nitroanilines of the formula III 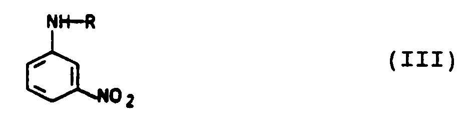 where R has the abovementioned meaning, with oleum.

## Revendications

1. Acides nitroaniline-sulfoniques de formule I 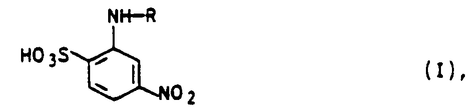 dans laquelle R signifie un groupement alkyle en C₁-C₄.

2. Procédé de préparation d'acides nitroaniline-sulfoniques selon la revendication 1, caractérisé en ce que l'on traite des nitroanilines de formule III 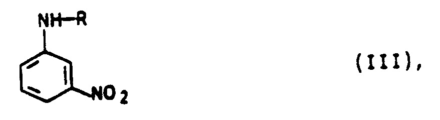 dans laquelle R a la signification susmentionnée, avec un oléum.
